(19) Europäisches Patentamt
European Patent Office
Office européen des brevets

(11) EP 1 945 265 B1

(12) EUROPEAN PATENT SPECIFICATION

(45) Date of publication and mention of the grant of the patent:
**11.07.2012 Bulletin 2012/28**

(21) Application number: **06808522.4**

(22) Date of filing: **13.11.2006**

(51) Int Cl.:
***A61K 45/06*** (2006.01) ***A61K 31/7068*** (2006.01)
***A61P 35/00*** (2006.01)

(86) International application number:
**PCT/GB2006/004230**

(87) International publication number:
**WO 2007/054731 (18.05.2007 Gazette 2007/20)**

(54) **ANTIPROLIFERATIVE COMBINATION COMPRISING CYC-682 AND A CYTOTOXIC AGENT**

ANTIPROLIFERATIVE KOMBINATION AUS CYC-682 UND EINEM ZYTOTOXISCHEN MITTEL

PRODUIT COMPOSE ANTIPROLIFERATIF COMPRENANT CYC-682 ET UN AGENT CYTOTOXIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **11.11.2005 GB 0523041**

(43) Date of publication of application:
**23.07.2008 Bulletin 2008/30**

(73) Proprietor: **Cyclacel Limited**
**London N1 7JQ (GB)**

(72) Inventors:
- **GREEN, Simon**
  **Perth and Kinross DD2 5DW (GB)**
- **FLEMING, Ian**
  **Angus DD7 7NT (GB)**
- **RAYMOND, Eric**
  **94880 Noiseau (FR)**

(74) Representative: **Clyde-Watson, Zöe et al**
**D Young & Co LLP**
**120 Holborn**
**London EC1N 2DY (GB)**

(56) References cited:
**EP-A1- 0 536 936** **WO-A-2005/053699**
**WO-A2-2005/000204**

- **DATABASE WPI Week 200105 Derwent Publications Ltd., London, GB; AN 2001-040935 XP002416553 & WO 00/67760 A1 (SANKYO CO LTD) 16 November 2000 (2000-11-16)**
- **DATABASE WPI Week 200052 Derwent Publications Ltd., London, GB; AN 2000-565332 XP002416554 & WO 00/48611 A1 (SANKYO CO LTD) 24 August 2000 (2000-08-24)**
- **WU M ET AL: "HIGH-RESOLUTION MAGNETIC RESONANCE IMAGING OF THE EFFICACY OF THE CYTOSINE ANALOGUE 1-[2-C-CYANO-2-DEOXY-BETA-D-ARABINO-PENTOF URANOSYLÜ N4-PALMITOYL CYTOSINE (CS-682) IN A LIVER-METASTASIS ATHYMIC NUDE MOUSE MODEL" CANCER RESEARCH, AMERICAN ASSOCIATION FOR CANCER RESEARCH, BALTIMORE, MD, US, vol. 63, no. 10, 15 May 2003 (2003-05-15), pages 2477-2482, XP001179127 ISSN: 0008-5472**
- **HANAOKA K ET AL: "ANTITUMOR ACTIVITY AND NOVEL DNA-SELF-STRAND-BREAKING MECHANISM OF CNDAC (1-(2-C-CYANO-2-DEOXY-BETA-D-ARABINO-PENTO FURANOSYL) CYTOSINE) AND ITS N4-PALMITOYL DERIVATIVE (CS-682)" INTERNATIONAL JOURNAL OF CANCER, NEW YORK, NY, US, vol. 82, no. 2, 1999, pages 226-236, XP001179126 ISSN: 0020-7136**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 945 265 B1

## Description

The present invention relates to a pharmaceutical combination suitable for the treatment of proliferative disorders.

## BACKGROUND TO THE INVENTION

The therapeutic use of pyrimidine nucleosides in the treatment of proliferative disorders has been well documented in the art. By way of example, commercially available antitumor agents of the pyrimidine series include 5-fluorouracil (Duschinsky, R., et al., J. Am. Chem. Soc., 79, 4559 (1957)), Tegafur (Hiller, SA., et al., Dokl. Akad. Nauk USSR, 176, 332 (1967)), UFT (Fujii, S., et al., Gann, 69, 763 (1978)), Carmofur (Hoshi, A., et al., Gann, 67, 725 (1976)), Doxyfluridine (Cook, A. F., et al., J. Med. Chem., 22, 1330 (1979)), Cytarabine (Evance, J. S., et al., Proc. Soc. Exp. Bio. Med., 106. 350 (1961)), Ancytabine (Hoshi, A., et al., Gann, 63, 353, (1972)) and Enocytabine (Aoshima, M., et al., Cancer Res., 36, 2726 (1976)).

Nucleoside analogues that show antimetabolic activity in cancer cells have been successfully used in the treatment of various human malignancies. Nucleosides such as 1-beta-D-arabinofuranosylcytosine (Ara-C), fludarabine and cladribine play an important role in the treatment of leukemias, while gemcitabine is extensively used in the treatment of many types of solid tumors. These compounds are metabolized in a similar manner to endogenous nucleosides and nucleotides. Active metabolites interfere with the *de novo* synthesis of nucleosides and nucleotides and/or inhibit DNA chain elongation after being incorporated into DNA strands, acting as chain terminators. Furthermore, nucleoside antimetabolites incorporated into DNA strands induce strand-breaks that may eventually result in induction of apoptosis.

Nucleoside antimetabolites target one or more specific enzyme(s) (Galmarini et al, Nucleoside analogues and nucleobases in cancer treatment. Lancet Oncol. 2002 Jul;3(7):415-24; Review). The mode of inhibitory action on target enzymes may differ between nucleoside antimetabolites, which have the same nucleoside base, such as Ara-C and gemcitabine. Although both nucleosides are phosphorylated by deoxycytidine kinase and are also good substrates of cytidine deaminase, only gemcitabine shows antitumor activity against solid tumors. This suggests that there are differences in the pharmacological activity of these nucleoside antimetabolites, which may reflect different modes of action on target molecules.

It was shown that dCK deficiency has been associated with resistance to Ara-C in various cell and animal models (Galmarini et al, In vivo mechanisms of resistance to cytarabine in acute myeloid leukaemia, Br J Haematol. 2002 Jun;117(4):860-8). Alterations in expression of the dCK gene or significant decrease in the activity of this enzyme in Ara-C-treated AML patients have also been correlated with clinical outcome. These data are consistent with the concept that intracellular phosphorylation of Ara-C by dCK is essential for cytotoxicity in cellular models and in patients. Deficiency of hENT1 in blast cell plasma membranes has also been suggested as a mechanism of cellular resistance to Ara-C. Other authors have suggested that mechanism of drug resistance to Ara-C are associated with increased levels of Ara-C catabolic enzymes such as CDA.

EP 536936 (Sankyo Company Limited) discloses various 2'-cyano-2'-deoxy-derivatives of 1-β-D-arabinofuranosylcytosine which have been shown to exhibit valuable anti-tumour activity. One particular compound disclosed in EP 536936 is 2'-cyano-2'-deoxy-$N^4$-palmitoyl-1-β-D-arabinofuranosylcytosine (referred to hereinafter as "CYC682"), this compound is currently under further investigation.

CYC682, also known as 1-(2-C-cyano-2-dioxy-β-D-arabino-pentofuranosyl)-$N^4$-palmitoyl cytosine (Hanaoka, K., et al, Int. J. Cancer, 1999:82:226-236; Donehower R, et al, Proc Am Soc Clin Oncol, 2000: abstract 764; Burch, PA, et al, Proc Am Soc Clin Oncol, 2001: abstract 364), is an orally administered novel 2'-deoxycytidine antimetabolite prodrug of the nucleoside CNDAC, 1-(2-C-Cyano-2-deoxy-β-D-arabino-pentafuanosyl)-cytosine.

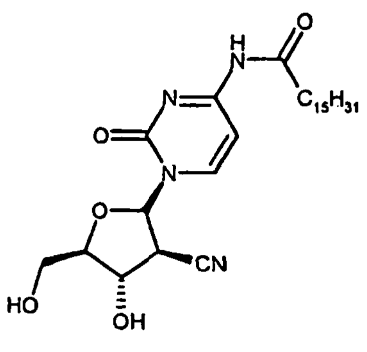

CYC682

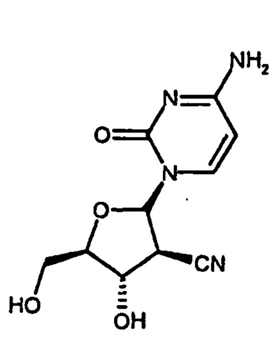

CNDAC

CYC682 has a unique mode of action over other nucleoside metabolites such as gemcitabine in that it has a spontaneous DNA strand breaking action, resulting in potent anti-tumour activity in a variety of cell lines, xenograft and metastatic cancer model (Hanaoka *et al,* 1999; Kaneko et al, 1997; Wu et al, 2003).

CYC682 has been the focus of a number of studies in view of its oral bioavailability and its improved activity over gemcitabine (the leading marketed nucleoside analogue) and 5-FU (a widely-used antimetabolite drug) based on preclinical data in solid tumours. Recently, investigators reported that CYC682 exhibited strong anticancer activity in a model of colon cancer. In the same model, CYC682 was found to be superior to either gemcitabine or 5-FU in terms of increasing survival and also preventing the spread of colon cancer metastases to the liver (Wu M, et al, Cancer Research, 2003:63:2477-2482). To date, phase I data from patients with a variety of cancers suggest that CYC682 is well tolerated in humans, with myelosuppression as the dose limiting toxicity.

It well established in the art that active pharmaceutical agents can often be administered in combination in order to optimise the treatment regime. For example, combinations comprising a CDK inhibitor and 1-(2-C-cyano-2-dioxy-β-D-arabino-pentofuranosyl)-N4-palmitoyl cytosine, or a metabolite thereof, and their use in the treatment of proliferative disorders are disclosed in WO 2005/053699 (Cyclacel Limited).

The present invention seeks to provide new combinations of known pharmaceutical agents that are particularly suitable for the treatment of proliferative disorders, especially cancer. More specifically, the invention relates to combinations comprising 2'-cyano-2'-deoxy-$N^4$-palmitoyl-1-β-D-arabinofuranosyl-cytosine, or a metabolite thereof which is 1-(2-C-Cyano-2-deoxy-β-D-arabino-pentafuranosyl)-cytosine, with classical cytotoxic drugs, selected from vinorelbine, docetaxel, cisplatin and oxaliplatin.

## STATEMENT OF THE INVENTION

A first aspect of the invention relates to a combination comprising 2'-cyano-2'-deoxy-$N^4$-palmitoyl-1-β-D-arabinofuranosyl-cytosine, or a metabolite thereof which is 1-(2-C-Cyano-2-deoxy-β-D-arabino-pentafuranosyl)-cytosine, or a pharmaceutically acceptable salt thereof, and a cytotoxic agent selected from: (a) vinorelbine; (b) docetaxel; and (c) a platinum antineoplastic agent selected from cisplatin and oxaliplatin.

Although 2'-cyano-2'-deoxy-$N^4$-palmitoyl-1-β-D-arabinofuranosyl-cytosine and the above-mentioned cytotoxic agents are well established in the art as individual therapeutic agents, there has been no suggestion that the specific combinations claimed in the present invention would be particularly effective in the treatment of cancer.

A second aspect relates to a pharmaceutical composition comprising a combination according to the invention and a pharmaceutically acceptable carrier, diluent or excipient.

A third aspect relates to the use of a combination according to the invention in the preparation of a medicament for treating a proliferative disorder.

Also referred to is a pharmaceutical product comprising (i) 2'-cyano-2'-deoxy-$N^4$-palmitoyl-1-β-D-arabinofuranosyl-cytosine, or a metabolite thereof as defined above, or a pharmaceutically acceptable salt thereof, and (ii) a cytotoxic agent selected from: (a) a vinca alkaloid; (b) a taxane; (c) a cytosine analogue; (d) an anthracycline; and (e) a platinum antineoplastic agent, as a combined preparation for simultaneous, sequential or separate use in therapy. In the claimed invention, the cytotoxic agent is restricted to the 4 compounds, selected from groups (a),(b) and (e), as defined in the first aspect above.

Another aspect relates to the use of 2"cyano-2'-deoxy-$N^4$-palmitoyl-1-β-D-arabinofuranosyl-cytosine, or a metabolite thereof which is 1-(2-C-Cyano-2-deoxy-β-D-arabino-pentafuranosyl)-cytosine, or a pharmaceutically acceptable salt thereof, in the preparation of a medicament for the treatment of a proliferative disorder, wherein said treatment comprises simultaneously, sequentially or separately administering a cytotoxic agent selected from (a) vinorelbine; (b) docetaxel; and (c) a platinum antineoplastic agent selected from cisplatin and oxaliplatin, to a subject.

Another aspect relates to the use of a cytotoxic agent as defined in the first aspect above in the preparation of a medicament for the treatment of a proliferative disorder, wherein said treatment comprises simultaneously, sequentially or separately administering to a subject 2'-cyano-2'-deoxy-$N^4$-palmitoyl-1-β-D-arabinofuranosyl-cytosine, or a metabolite thereof as defined above, or a pharmaceutically acceptable salt thereof.

Another aspect relates to the use of 2'-cyano-2'-deoxy-$N^4$-palmitoyl-1-β-D-arabinofuranosyl-cytosine, or a metabolite thereof as defined above, or a pharmaceutically acceptable salt thereof, and a cytotoxic agent as defined in the first aspect above in the preparation of a medicament for treating a proliferative disorder.

Another aspect relates to the use of a cytotoxic agent as defined in the first aspect above, in the preparation of a medicament for the treatment of a proliferative disorder, wherein said medicament is for use in combination therapy with 2'-cyano-2'-deoxy-$N^4$-palmitoyl-1-β-D-arabinofuranosyl-cytosine, or a metabolite thereof as defined above, or a pharmaceutically acceptable salt thereof.

Another aspect relates to the use of 2'-cyano-2'-deoxy-$N^4$-palmitoyl-1-β-D-arabinofuranosyl-cytosine, or a metabolite thereof as defined above, or a pharmaceutically acceptable salt thereof, in the preparation of a medicament for the treatment of a proliferative disorder, wherein said medicament is for use in combination therapy with a cytotoxic

agent as defined in the first aspect above.

Another aspect of the invention relates to the use of 2'-cyano-2'-dcoxy-$N^4$-palmitoyl-1-β-D-arabinofuranosyl-cytosine, or a metabolite thereof as defined above, or a pharmaceutically acceptable salt thereof, in the preparation of a medicament for the treatment of a proliferative disorder, wherein said medicament is for use in pretreatment therapy with a cytotoxic agent as defined in the first aspect above.

## DETAILED DESCRIPTION

The preferred embodiments set out below are applicable to all the above-mentioned aspects of the invention.

One preferred embodiment of the present invention relates to a combination comprising 2'-cyano-2'-deoxy-$N^4$-palmitoyl-1-β-D-arabinofuranosyl-cytosine (CYC682), or a metabolite thereof which is 1-(2-C-Cyano-2-deoxy-β-D-arabino-pentafuranosyl)-cytosine, and a cytotoxic agent selected from oxaliplatin and docetaxel.

Another aspect of the present invention relates to a pharmaceutical composition comprising CYC682, or a metabolite thereof which is 1-(2-C-Cyano-2-deoxy-β-D-arabino-pentafuranosyl)-cytosine, and a cytotoxic agent selected from (a) vinorelbine; (b) docetaxel; and (c) a platinum antineoplastic agent selected from cisplatin and oxaliplatin.

Another preferred embodiment of the present invention relates to a pharmaceutical composition comprising CYC682, or a metabolite thereof which is 1-(2-C-Cyano-2-deoxy-β-D-arabino-pentafuranosyl)-cytosine, and a cytotoxic agent selected from oxaliplatin and docetaxel.

The metabolite is 1-(2-C-Cyano-2-deoxy-β-D-arabino-pentafuranosyl)-cytosine, also known as "CNDAC".

Another aspect relates to a pharmaceutical product comprising the combination of the present invention for use in the treatment of a proliferative disorder, wherein the disorder is preferably cancer.

A further aspect of the present invention relates to a pharmaceutical product comprising CYC682, or a metabolite thereof as defined above, and a cytotoxic agent as defined above in the first aspect as a combined preparation for simultaneous, sequential or separate use in therapy.

One embodiment of the present invention relates to a pharmaceutical product comprising CYC682, or a metabolite thereof as defined above, and a cytotoxic agent as defined in the first aspect above, as a combined preparation for simultaneous, sequential or separate use in therapy.

As used herein, "simultaneously" is used to mean that the two agents are administered concurrently, whereas the term "in combination" is used to mean that they are administered, if not simultaneously, then "sequentially" with a timeframe that they are able to act therapeutically within the same timeframe. Thus, administration "sequentially" may permit one agent to be administered within 5 minutes, 10 minutes or a matter of hours after the other, provided that they are both concurrently present in therapeutic amounts. The time delay between administration of the components will vary depending on the exact nature of the components, the interaction therebetween and their respective half-lives.

In contrast to "in combination" or " sequentially", "separately" is used herein to mean that the gap between administering one agent and the other is significant i.e. the first administered agent may no longer be present in the bloodstream in a therapeutically effective amount when the second agent is administered.

In the following pages of the description, references to a metabolite and to cytotoxic agents in the framework of the claimed invention should always be interpreted as referring to the metabolite and cytotoxic agents as defined in the first aspect of the invention above.

In one preferred embodiment, the pharmaceutical product comprises administering the cytotoxic agent sequentially or separately prior to the CYC682, or metabolite thereof.

In another preferred embodiment, the pharmaceutical product comprises administering the CYC682, or metabolite thereof, sequentially or separately prior to the cytotoxic agent.

In one preferred embodiment, the CYC682 (or metabolite thereof) is administered at least 1 hour, or at least 4 hours, or at least 8 hours, 12, hours, 24 hours or 48 hours prior to the cytotoxic agent.

In another preferred embodiment, the cytotoxic agent is administered at least 1 hour, or at least 4 hours, or at least 8 hours, 12, hours, 24 hours or 48 hours prior to the CYC682 (or metabolite thereof).

In another preferred embodiment, the cytotoxic agent and CYC682 (or metabolite thereof) are administered concurrently.

In one highly preferred embodiment, the CYC682 (or metabolite thereof) and the cytotoxic agent are administered in accordance with the regimen set forth in Figure 2, i.e., the CYC682 is administered for 48 hours, followed by administration of the cytotoxic agent for 24 hours, or the cytotoxic drug is administered for 24 hours, followed by administration of CYC682 for 48 hours, or the cytotoxic agent and the CYC682 are administered concurrently for 24, 48 or 72 hours. The dosing regimen can be repeated as necessary, preferably with treatment-free periods in between each treatment cycle.

In a preferred embodiment, the CYC682, or metabolite thereof, and the cytotoxic agent are each administered in a therapeutically effective amount with respect to the individual components.

In another preferred embodiment, the CYC682, or metabolite thereof, and the cytotoxic agent are each administered in a sub-therapeutic amount with respect to the individual components.

The term "sub-therapeutic amount" means an amount that is lower than that typically required to produce a therapeutic effect with respect to treatment with CYC682 alone or the cytotoxic agent alone.

A further aspect relates to the use of the combination of the present invention in the preparation of a medicament for treating a proliferative disorder.

As used herein the phrase "preparation of a medicament" includes the use of one or more of the above described components directly as the medicament or in any stage of the manufacture of such a medicament.

Another aspect of the invention relates to the use of CYC682, or a metabolite thereof, in the preparation of a medicament for the treatment of a proliferative disorder, wherein said treatment comprises simultaneously, sequentially or separately administering a cytotoxic agent selected from (a) vinorelbine; (b) docetaxel; and (c) a platinum antineoplastic agent selected from cisplatin and oxaliplatin to a subject.

One particularly preferred embodiment relates to the use of CYC682, or a metabolite thereof, in the preparation of a medicament for the treatment of a proliferative disorder, wherein said treatment comprises simultaneously, sequentially or separately administering a cytotoxic agent selected from oxaliplatin and docetaxel to a subject.

Another aspect relates to the use of a cytotoxic agent as defined above, in the preparation of a medicament for the treatment of a proliferative disorder, wherein said medicament is for use in combination therapy with CYC682, or a metabolite thereof as defined above. In one preferred embodiment, the therapy can be pretreatment therapy.

One particularly preferred embodiment relates to the use of a cytotoxic agent as defined above, in the preparation of a medicament for the treatment of a proliferative disorder, wherein said medicament is for use in combination therapy with CYC682, or a metabolite thereof as defined above. In one preferred embodiment, the therapy can be pretreatment therapy.

Another aspect relates to the use of CYC682, or metabolite thereof, in the preparation of a medicament for the treatment of a proliferative disorder, wherein said medicament is for use in combination therapy with a cytotoxic agent selected from (a) vinorelbine; (b) docetaxel; and (c) a platinum antineoplastic agent selected from cisplatin and oxaliplatin. In one preferred embodiment, the therapy can be pretreatment therapy.

One particularly preferred embodiment relates to the use of CYC682, or metabolite thereof, in the preparation of a medicament for the treatment of a proliferative disorder, wherein said medicament is for use in combination therapy with a cytotoxic agent selected from oxaliplatin and docetaxel. Again, in one highly preferred embodiment, the therapy can be pretreatment therapy.

As used herein, the term "combination therapy" refers to therapy in which the cytotoxic agent and CYC682 (or metabolite thereof) are administered, if not simultaneously, then sequentially within a timeframe that they both are available to act therapeutically within the same time-frame.

As used herein, the term "pretreatment therapy" or "pretreated" means a regimen in which one agent is administered prior to, either separately or sequentially, the second agent. Preferably, the second agent is administered at least 2 hours after the administration of the first agent. More preferably, the second agent is administered at least 4 hours, or more preferably at least 6 or 8 hours, after the administration of the first agent. Even more preferably, the second agent is administered at least 12 hours, or more preferably at least 18 or 24 hours, after the administration of the first agent.

Preferably, CYC682 or metabolite thereof, and the cytotoxic agent interact in a synergistic manner. As used herein, the term "synergistic" means that CYC682 and the cytotoxic agent produce a greater effect when used in combination than would be expected from adding the individual effects of the two components. Advantageously, a synergistic interaction may allow for lower doses of each component to be administered to a patient, thereby decreasing the toxicity of chemotherapy, whilst producing and/or maintaining the same therapeutic effect. Thus, in a particularly preferred embodiment, each component can be administered in a sub-therapeutic amount.

In another preferred embodiment, the CYC682 or metabolite thereof, and the cytotoxic agent interact in a manner so as to alleviate or eliminate adverse side effects associated with use of the individual components in monotherapy, or associated with their use in known combinations.

In one preferred embodiment of the invention, the cytotoxic agent is a platinum antineoplastic selected from cisplatin and oxaliplatin.

In one highly preferred embodiment, the platinum antineoplastic is oxaliplatin.

Oxaliplatin is a cytotoxic agent containing a platinum atom complexed with oxalate and diaminocyclohexane (DACH). The bulky DACH is thought to contribute greater cytotoxicity than cisplatin and carboplatin (Wiseman LR, Adkins JC, Plosker GL, et al. Oxaliplatin: a review of its use in the management of metastatic colorectal cancer. Drugs Aging 1999;14(6):459-75). The exact mechanism of action of oxaliplatin is not known, although it is known to be cell-cycle-phase nonspecific. Oxaliplatin forms reactive platinum complexes which are believed to inhibit DNA synthesis by forming interstrand and intrastrand cross-linking of DNA molecules. Oxaliplatin is not generally cross-resistant to cisplatin or carboplatin, possibly due to the DACH group and resistance to DNA mismatch repair (Wiseman LR *et al, ibid*; Misset JL, Bleiberg H, Sutherland W, et al, Oxaliplatin Clinical Activity: A Review; Critical Reviews in Oncology-Hematology

2000;35(2):75-93). Preclinical studies have shown oxaliplatin to be synergistic with fluorouracil and SN-38, the active metabolite of irinotecan (Cvitkovic E, Bekradda M. Oxaliplatin: A New Therapeutic Option in Colorectal Cancer; Semin Oncol 1999;26(6):647-62). Oxaliplatin is also a radiation-sensitizing agent (Freyer G, Bossard N, Romestaing P, et al, Oxaliplatin (OXA), 5-fluorouracil (5FU), L-folinic acid (FA) and concomitant irradiation in patients with rectal cancer: A phase 1 study; Proc Am Soc Clin Oncol 2000;19:260a; Carraro S, Roca E, Cartelli C, et al, Oxaliplatin (OXA), 5-fluorouracil (5-Fu) and leucovorin (LV) plus radiotherapy in unresectable rectal cancer (URC): Preliminary results; Proc Am Soc Clin Oncol 2000;19:291a).

Oxaliplatin is approved for use in combination with 5-fluorouracil (5-FU) and folinic acid (FA) in adjuvant treatment of stage III (Duke's C) colon cancer after complete resection of primary tumor, and in the treatment of metastatic colorectal cancer.

Preferably, where the cytotoxic agent is oxaliplatin, the cytotoxic agent and CYC682 (or metabolite thereof) are administered separately or sequentially, more preferably, sequentially.

In one particularly preferred embodiment, where the cytotoxic agent is oxaliplatin, the CYC682 (or metabolite thereof) is administered prior to the cytotoxic agent, i.e. preferably, the subject is pretreated with CYC682.

In another particularly preferred embodiment, where the cytotoxic agent is oxaliplatin, the cytotoxic agent is administered prior to the CYC682 (or metabolite thereof), i.e. preferably, the subject is pretreated with oxaliplatin.

In another particularly preferred embodiment, the cytotoxic agent is cisplatin.

The compound *cis*-diamminedichloroplatinum (II), commonly referred to as cisplatin or *cis*-DDP, is a known anticancer agent which is widely used in the clinic, particularly in the treatment of testicular cancer. The molecular structure is relatively simple and consists of two chlorine ligands and two $NH_3$ ligands situated in the *cis* position, forming a tetragonal (square) planar structure around a central platinum atom. Cisplatin exists as an electroneutral, four-coordinate platinum complex. However, studies have shown that the dihydrated (active) form promotes binding to DNA.

Cisplatin is generally administered into the bloodstream intravenously as a sterile saline solution. Due to the high chloride concentration in the bloodstream, the drug remains intact in its neutral form. It then enters the cell by diffusion where it undergoes hydrolysis as a result of the much lower intracellular chloride concentration. Hydrolysis converts the neutral molecule into the active hydrated complex in which both chloride ligands are replaced by water molecules to generate a positively charged species. The active form is a bifunctional electrophilic agent which is able to undergo nucleophilic substitution with DNA base pairs.

Cisplatin has biochemical properties similar to that of bifunctional alkylating agents, producing interstrand, intrastrand and monofunctional adduct cross-linking in DNA. The most prevalent form is the 1,2-intrastrand crosslink. In this adduct, the platinum is covalently bound to the N7 position of adjacent purine bases. As a consequence, the DNA is unwound and bent towards the major groove. Other platinum-DNA adducts include monofunctional and 1,3- and longer range intrastrand, interstrand and protein-DNA crosslinks.

Studies have shown that most adducts involve guanine residues as these offer three sites for hydrogen bonding with cytosine, thereby leading to greater stability compared to the two hydrogen bonds which are possible between adenine and thymine. The formation of a cisplatin-DNA adduct distorts the DNA structure which in turn leads to disruption of replication and transcription. In addition, the formation of a cisplatin-DNA adduct disrupts the ability of the cells to repair themselves, either by blocking and slowing down repair proteins, or negatively altering the function of nucleotide excision repair (NER) proteins, specifically XPA.

Cisplatin is approved for use in the treatment of metastatic, non-seminomatous germ cell carcinoma, advanced stage and refractory ovarian carcinoma, lung cancer, cervical cancer, advanced stages and refractory bladder carcinoma and squamous cell carcinoma of head and neck. Cisplatin is also indicated in combination with other antineoplastic agents for the treatment of metastatic testicular tumours. The combination of cisplatin, vinblastine and bleomycin is reported to be highly effective.

In one preferred embodiment, the combination comprises CNDAC (i.e the metabolite of CYC682) and cisplatin. Preferably, for this embodiment, the cisplatin is administered concurrently with, or prior to, the CNDAC. More preferably, the cisplatin is administered prior to the CNDAC.

In one preferred embodiment, the cytotoxic agent is the taxane docetaxel. The taxanes are a class of alkaloids derived from plants of the genus *Taxus* (yews). The principal mechanism of the taxane class is inhibition of microtubule function, which in turn inhibits cell division.

Docetaxel is an anticancer drug of the taxane family which is prepared by hemisynthesis from the renewable needles of the European yew tree (*Taxus baccata*). Docetaxel is widely used in the clinic to treat a number of cancers including locally advanced or metastatic breast cancer, locally advanced or metastatic non-small cell lung cancer and hormone refractory prostate cancer. The mechanism of action is based upon disruption of the microtubular network which plays an essential role in cell division. More recent studies have focussed on the use of docetaxel in the first line treatment of non-small cell lung cancer alone or in combination, head and neck cancer, breast cancer, gastric cancer, prostate cancer and ovarian cancer.

Preferably, where the cytotoxic agent is docetaxel, the cytotoxic agent and CYC682 (or metabolite thereof) are

administered separately or sequentially, more preferably, sequentially.

In one particularly preferred embodiment, where the cytotoxic agent is docetaxel, the cytotoxic agent is administered prior to the CYC682 (or metabolite thereof), i.e. preferably, the subject is pretreated with docetaxel.

In another preferred embodiment, the cytotoxic agent is the vinca alkaloid, vinorelbine.

The vinca alkaloids are a group of indole-indoline dimers derived from the periwinkle plant, *Catharanthus roseus* (also *Vinca rosea, Lochnera rosea,* and *Ammocallis rosea*). They are known to inhibit polymerization of tubulin into microtubules, thus blocking spindle formation and arresting cells in metaphase. Vinca alkaloids include vinblastine, vincamine, vinorelbine, vincristine, vindesine and vinpocetine.

In one highly preferred embodiment, the cytotoxic agent is vinorelbine.

Vinorelbine is approved for use as a single agent or in combination for the first line treatment of stage 3 or 4 non-small cell lung cancer and in the treatment of advanced breast cancer stage 3 and 4 relapsing after, or refractory to, an anthracycline containing regimen.

In one preferred embodiment, the combination of the invention comprises CNDAC (i.e. the the metabolite of CYC682) and vinorelbine as the cytotoxic agent. For this embodiment, the vinorelbine may be administered prior to, or concurrently with, or after the CNDAC.

## PROLIFERATIVE DISORDER

The term "proliferative disorder" is used herein in a broad sense to include any disorder that requires control of the cell cycle, for example cardiovascular disorders such as restenosis and cardiomyopathy, auto-immune disorders such as glomerulonephritis and rheumatoid arthritis, dermatological disorders such as psoriasis, anti-inflammatory, anti-fungal, antiparasitic disorders such as malaria, emphysema and alopecia. In these disorders, the compounds of the present invention may induce apoptosis or maintain stasis within the desired cells as required.

In respect of all of the above aspects and embodiments, preferably the proliferative disorder is cancer, more preferably, colon cancer.

In another preferred embodiment, the cancer is lung cancer, more preferably non-small cell lung cancer.

In another preferred embodiment, when the cytotoxic agent is oxaliplatin, the proliferative disorder is colorectal cancer.

In yet another preferred embodiment, when the cytotoxic agent is docetaxel, the proliferative disorder is breast, lung or prostate cancer.

In yet another preferred embodiment, when the cytotoxic agent is cisplatin, the proliferative disorder is ovarian cancer, lung cancer, cervical cancer, testicular cancer, bladder cancer or squamous cell carcinoma of head and neck.

In yet another preferred embodiment, when the cytotoxic agent is vinorelbine, the proliferative disorder is breast cancer or lung cancer. More preferably, the proliferative disorder is non-small cell lung cancer.

## PHARMACEUTICAL COMPOSITIONS

In a particularly preferred embodiment, the pharmaceutical product of the invention is in the form of a pharmaceutical composition comprising a pharmaceutically acceptable carrier, diluent or excipient.

Even though the compounds of the present invention (including their pharmaceutically acceptable salts, esters and pharmaceutically, acceptable solvates) can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients", 2nd "Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

## SALTS/ESTERS

The agents of the present invention can be present as salts or esters, in particular pharmaceutically acceptable salts or esters.

Pharmaceutically acceptable salts of the agents of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C1-C4)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C1-C4)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

## ENANTIOMERS/TAUTOMERS

The invention also includes where appropriate all enantiomers and tautomers of the agents. The man skilled in the art will recognise compounds that possess optical properties (one or more chiral carbon atoms) or tautomeric characteristics. The corresponding enantiomers and/or tautomers may be isolated/prepared by methods known in the art.

## STEREO AND GEOMETRIC ISOMERS

Some of the agents of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the agent or pharmaceutically acceptable salts thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as 2H, 3H, 13C, 14C, 15N, 170, 180, 31P, 32P, 35S, 18F and 36Cl, respectively. Certain isotopic variations of the agent and pharmaceutically acceptable salts thereof, for example, those in which a radioactive isotope such as 3H or 14C is incorporated, are useful in drug and/or substrate tissue distribution studies. Tritiated, i.e., 3H, and carbon-14, i.e., 14C, isotopes are particularly preferred for their ease of preparation and detectability. Further, substitution with isotopes such as deuterium, i.e., 2H, may afford certain therapeutic advantages resulting from greater metabolic stability, for example, increased *in vivo* half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

## SOLVATES

The present invention also includes solvate forms of the agents of the present invention. The terms used in

the claims encompass these forms.

## POLYMORPHS

The invention furthermore relates to agents of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

## PRODRUGS

Furthermore, reference is made herein to agents in prodrug form. Such prodrugs are generally compounds wherein one or more appropriate groups have been modified such that the modification may be reversed upon administration to a human or mammalian subject. Such reversion is usually performed by an enzyme naturally present in such subject, though it is possible for a second agent to be administered together with such a prodrug in order to perform the reversion *in vivo*. Examples of such modifications include ester (for example, any of those described above), wherein the reversion may be carried out be an esterase etc. Other such systems will be well known to those skilled in the art.

## ADMINISTRATION

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 2000 mg and more preferably from 50-1000 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 500 mg, of active ingredient per dose.

Compositions may be formulated in unit dosage form, i.e., in the form of discrete portions containing a unit dose, or a multiple or sub-unit of a unit dose.

In a particularly preferred embodiment, the combination or pharmaceutical composition of the invention is administered intravenously.

## DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Depending upon the need, the agent may be administered at a dose of from 0.1 to 30 mg/kg body weight, such as from 2 to 20 mg/kg, more preferably from 0.1 to 1 mg/kg body weight.

By way of guidance, the cytotoxic agent is typically administered in accordance with a physician's direction at dosages between the approved dosages for said cytotoxic agent. Said dosages are available from the Summary of Product Characteristics for each agent which may be obtained from the manufacturer or from the literature e.g. www.emea.eu.int/htms/human/epar/a-zepar.htm.

By way of guidance, CYC682 is typically administered in accordance to a physicians direction at dosages

between 0.05 to 5g for an adult human patient. Preferably, the dosage is between 1 and 120 $mg/m^2$ body surface orally. The doses can be given 5 days a week for 4 weeks, or 3 days a week for 4 weeks. Dosages and frequency of application are typically adapted to the general medical condition of the patient and to the severity of the adverse effects caused, in particular to those caused to the hematopoietic, hepatic and to the renal system. The total daily dose of CYC682 can be administered as a single dose or divided into separate dosages preferably administered two, three or four time a day.

Preferably, the cytotoxic agent is administered at least 2 hours before the administration of the CYC682, or metabolite thereof. More preferably, the cytotoxic agent is administered at least 4 hours, or more preferably at least 6 or 8 hours, before the administration of the CYC682, or metabolite thereof. Even more preferably, the cytotoxic agent is administered at least 12 hours, or more preferably at least 18 or 24 hours, before the administration of the CYC682, or metabolite thereof.

In another preferred embodiment, the cytotoxic agent is administered at least 2 hours after the administration of the CYC682, or metabolite thereof. More preferably, the cytotoxic agent is administered at least 4 hours, or more preferably at least 6 or 8 hours, after the administration of the CYC682, or metabolite thereof. Even more preferably, the cytotoxic agent is administered at least 12 hours, or more preferably at least 18 or 24 hours, after the administration of the CYC682, or metabolite thereof.

The present invention is further described by way of example, and with reference to the following figures, wherein:

Figure 1 shows the effects of CYC682 and CNDAC against a panel of cell lines (% survival against concentration in μM).

Figure 2 shows sequences 1, 2 and 3 evaluating the effects of CYC682 based combination. Readout was done immediately after exposure H72 after exposition to drugs.

Figure 3 shows isobolograms showing the interaction of CYC682 and docetaxel in the human COLO205 and HCT116 cancer cell lines.

Figure 4 (Reference) shows isobolograms showing the interaction of CYC682 and gemcitabine in the human COLO205 and HCT116 cancer cell lines.

Figure 5 (Reference) shows isobolograms showing the interaction of CYC682 and doxorubicin in the human COLO205 and HCT116 cancer cell lines.

Figure 6 shows isobolograms showing the interaction of CYC682 and oxaliplatin in the human COLO205 and HCT116 cancer cell lines.

## EXAMPLES

As with Figures 4 and 5, any part of the examples referring to other cytotoxic agents than the four agents claimed, is to be interpreted as comparative or reference material to the claimed invention. For instance in that sense, drug-combination studies have been marked with an asterisk.

### Materials & Methods

Doxorubicin and the tissue culture reagents are supplied by Sigma Aldrich. CYC682 was supplied by Cyclacel Ltd. (Dundee UK). Docetaxol (Taxotere®, Aventis) was used as clinical formulation. Gemcitabine was supplied by Lilly. Oxaliplatin was supplied by Sanofi. Cisplatin and vinorelbine were obtained from Sigma Aldrich

Preparation of CYC682 (in accordance with EP 536936)

CYC682 was prepared in accordance with the methodology described in Examples 1 and 2 of EP 536936 in the name of Sankyo Company Limited.

Cell lines

All cell lines were obtained from the ATCC (Rockville, MD). Cells were grown as monolayers in RPMI medium supplemented with 10% fetal calf serum (Invitrogen, Cergy-Pontoise, France), 2 mM glutamine, 100 units/ml penicillin and 100 μg/ml streptomycin. All cells were split twice a week using trypsin/EDTA (0.25% and 0.02%, respectively; Invitrogen, Cergy-Pontoise, France) and seeded at a concentration of 2.5 x $10^4$ cells/ml. All cell lines were tested regularly

for *Mycoplasma* contamination by PCR using a Stratagene kit (La Jolla, CA).

*In vitro* growth inhibition assay (MTT assay)

The MTT assay was carried out as described previously (Hansen MB, Nielsen SE, Berg K. Re-examination and further development of a precise and rapid dye method for measuring cell growth/cell kill. J Immunol Methods. 1989 May 12;119(2):203-10). In brief, cells were seeded in 96-well tissue culture plates at a density of 2 x $10^3$ cells/well. Cell viability was determined after a 120-hour incubation, by the colorimetric conversion of yellow, water-soluble tetrazolium MTT (3-[4,5-dimethylthiazol-2-yl]-2,5-diphenyltetrazolium bromide; Sigma, Saint-Quentin Fallavier, France), into purple, water-insoluble formazan. This reaction is catalyzed by mitochondrial dehydrogenases and is used to estimate the relative number of viable cells (Mosmann, T, Rapid colorimetric assay for cellular growth and survival: application to proliferation and cytotoxicity assays. J Immunol Methods. 1983 Dec 16;65(1-2):55-63). Cells were incubated with 0.4 mg/ml MTT for 4 hours at 37°C. After incubation, the supernatant was discarded, the cell pellet was resuspended in 0.1 ml of DMSO and the absorbance was measured at 560 nm using a microplate reader (Dynatech, Michigan). Wells with untreated cells or with drug-containing medium without cells were used as positive and negative controls, respectively. Growth inhibition curves were plotted as a percentage of untreated control cells.

Single agent study

The cells were seeded at 2 x $10^3$ cells/well in 96-well plates and treated 24 hours later with increasing concentrations of CYC682. After incubation times of 1 hour, 24 hours or 48 hours, the cells were washed and post-incubated in drug-free medium for 72 hours. Growth inhibition was then determined by the MTT assay.

Simultaneous exposure of CYC682 with other drugs

For simultaneous drug exposure, cells were seeded at 2 x $10^3$ cells/well in 96-well plates and treated 24 hours later with increasing concentrations of CYC682 alone or with various concentrations of another drug corresponding to the $IC_{20}$, $IC_{40}$ or $IC_{60}$ values. After approximately four doubling times (120 hours), the growth inhibitory effect was measured by the MTT assay.

Sequential exposure to CYC682 and other drugs.

Cells were seeded at 2 x $10^3$ cells/well in 96-well plates and allowed to grow for 24 hour. Cells were then exposed to various concentrations of the first drug for 1 hour/24 hours/ 48 hours, the drug was removed, the cells were washed and the second drug was added. After additional drug exposure, the second drug was removed, the cells were washed and incubated in drug-free medium for 72 hours. Growth inhibition was then determined by the MTT assay.

CNDAC Combinations

Experiments in the NSCLC cell lines H1299 or RERF-LC-MA were performed in 96-well plates, with cells seeded at a density of 3,000/well, in DMEM containing 10% (v/v) FCS. Stock solutions of all drugs were made up in dimethylsulfoxide (DMSO), with the exception of gemcitabine, which was dissolved in 0.9% (w/v) sterile saline solution.

For experimental assessment of potential synergistic interactions, the concomitant treatment regime involved simultaneous treatment of cells with CNDAC and the other agent under investigation for 72 h, alongside suitable controls of cells treated with the individual compounds alone for 72 hr. In the sequential treatment regimes, one drug was added to the cells 2 h after plating, and left for 24 h. Media was then aspirated, replaced with fresh media containing the second drug and incubated for 72 h. The two individual treatment controls for the sequential treatment regime involved substituting one of the drug treatments with drug-free media. After drug treatment, the number of cells in each well was estimated by incubating for 1 h in media containing 10% alamar blue (Roche, Lewes, East Sussex, U.K.) and measuring the absorbance at 488-595 nm. Drug interactions were analysed using the Calcusyn software package (BioSoft, Cambridge, U.K.) described below. A Combination Index (C.I.) of 1 indicates an additive drug interaction, whereas a C.I. greater than 1 is antagonistic and a score lower than 1 is synergistic.

Statistical analysis and determination of synergistic activity

Effects of drug combinations were evaluated using the Chou and Talalay method which is based on the median-effect principle (Chou TC, Talalay P. Quantitative analysis of dose-effect relationships: the combined effects of multiple drugs or enzyme inhibitors. Adv Enzyme Regul. 1984;22:27-55). This involves plotting dose-effect curves for each drug

and for multiple diluted, fixed-ratio combinations, using the equation: $f_a / f_u = (C / C_m)^m$, where $f_a$ is the cell fraction affected by the drug concentration C (e.g., 0.9 if cell growth is inhibited by 90%), $f_u$ is the unaffected fraction, C is the drug concentration, $IC_{50}$ the concentration required for a half-maximal effect (i.e., 50% inhibition of cell growth), and m is the signioidicity coefficient of the concentration-effect curve. On the basis of the slope of the curve for each drug in a combination, it can be determined whether the drugs have mutually nonexclusive effects (e.g., independent or interactive modes of action).

The combination index (CI) is then determined by the equation:

$$CI = [(C)_1/(C_x)_1] + [(C)_2 / (C_x)_2] + [\alpha (C)_1 (C)_2 / (C_x)_1 (C_x)_2],$$

where $(Cx)_1$ is the concentration of drug 1 required to produce an x percent effect of that drug alone; and $(C)_1$, the concentration of drug 1 required to produce the same x percent effect in combination with $(C)_2$. If the mode of action of the drugs is mutually exclusive or nonexclusive, then $\alpha$ is 0 or 1, respectively. CI values will be calculated with this equation using different values of $f_a$ (i.e., for different degrees of cell growth inhibition). CI values of <1 indicate synergy, the value of 1 indicates additive effects, and values >1 indicate antagonism. Data were analyzed on an IBM-PC computer using concentration-effect analysis for microcomputer software (Biosoft, Cambridge, UK). For statistical analysis and graphs we will use Instat and Prism software (GraphPad, San Diego, USA). The dose-effect relationships for the drugs tested, alone or in paired combinations, were subjected to median-effect plot analysis to determine their relative potency ($IC_{50}$), shape (m), and conformity (r) in each selected cell line. As stated above, the $IC_{50}$ and m values were respectively used to calculate synergism and antagonism on the basis of the CI equation. Results were expressed as the mean $\pm$ standard deviation of at least 3 experiments performed in duplicate. In each experiment, cells were exposed to the paired combinations for 48 hours as described above. Means and standard deviations were compared using Student's *t*-test (two-sided *p* value).

**Results**

<u>Single agent studies</u>

*Antiproliferative effects of CYC682 and CNDAC given as single agent in a panel of human cancer cell lines*

Figure 1 shows the effects of CYC682 and CNDAC in a panel of cell lines. Each point is the average of at least 3 individual experiments, each done in duplicate. IC50s for 48 hour exposure are shown in Table 1. 24 hour exposure to CYC682 and CNDAC was tested and found to be not sufficient for CNDAC cytotoxicity. A 48-hour exposure was shown to be optimal to observe the antiproliferative effects of CYC682 in the most sensitive human cancer cell lines. Each point is the average of at least 3 individual experiments each performed in duplicate. CYC682 displayed cytotoxic effects against several human cancer cell lines, HCT116 being the most sensitive cancer cell line.

*Comparison of CYC682 cytotoxicity with other anticancer drugs*

A comparison was made between the cytotoxic effects of CYC682 and those of several anticancer drugs including oxaliplatin, cisplatin, doxorubicin, gemcitabine, vinorelbine, 5FU and Ara-C (Table 1). The data show that CYC682 displays antiproliferative activity at micromolar concentrations in most cancer cell lines and that its profile differs from that of classical anticancer agents such as oxaliplatin and cisplatin, as well as that of closely related antimetabolites such as cytarabine and gemcitabine. This suggests that mechanism(s) of action and resistance to CYC682 might, at least in part, be different from those of Ara-C and gemcitabine in human cancer cells.

**Drug-combination studies**

Combinations of CYC682 or CNDAC with cytotoxic agents marked with an asterisk (*) are included as reference examples only.

The effect of sequential and simultaneous exposure (Figure 2) to CYC682 with oxaliplatin, doxorubicin, docetaxel and gemcitabine was studied in two sensitive colon cancer cell lines - COLO205 and HCT116 using combination indices that represent an affected fraction for the concentration of drugs corresponding to IC50 as previously described by Chou and Talalay.

*Docetaxel-CYC682 combinations*

As shown in Figure 3, a synergistic effect was observed when docetaxel was given prior to CYC682 in both cell lines.

*Gemcitabine*-CYC682 combinations*

The sequence of gemcitabine followed by CYC682 was synergistic in both cell lines (Reference Figure 4). Synergism between CYC682 and gemcitabine suggest that although closely related, those two compounds may have distinct mechanisms of action in cancer cells.

*Doxorubicin*-CYC682 combinations*

Combinations of CYC682 with doxorubicin were synergistic at high concentrations using sequential exposure (Reference Figure 5).

*Oxaliplatin-CYC682 combinations*

Combinations of CYC682 with oxaliplatin led to synergistic activity when CYC682 was administered prior to or after oxaliplatin in HCT116 cells (Figure 6). In COLO205 cells, synergy was obtained when CYC682 was given prior to oxaliplatin.

*CNDAC Combinations*

CNDAC was tested in combination with doxorubicin*, cisplatin, gemcitabine* or docetaxel and the results suggest that all of these combinations generate synergistic drug interactions (Table 3).
CNDAC was also tested in combination with vinorelbine in H1299 cells, and the results indicate that this combination generates synergy with all three of the treatment regimes tested (Table 4).
In H1299 cells, CNDAC and doxorubicin* generated synergy at ED50 (when 50% of the cell population has been killed) with doxorubicin pre-treatment and concomitant pre-treatment regimes.
At ED50, CNDAC and cisplatin generated synergy with cisplatin pre-treatment and weak synergy with concomitant treatment.
CNDAC and gemcitabine* were tested in a concomitant treatment regime, which generated synergy at ED50.

**Discussion**

The data presented above show that CYC682 exhibits cytotoxic activity against a broad range of human cancer cell lines, HCT116 being the most sensitive. CYC682 and CNDAC display specific spectra of activity that differ from classical metabolites such as cytarabine, gemcitabine, 5FU and other cytotoxic agents (oxaliplatin, cisplatin, doxorubicin, vinorelbine, docetaxel).
Combination studies demonstrated synergistic effects when CYC682 (or CNDAC) was combined with drugs such as oxaliplatin, gemcitabine, docetaxel, vinorelbine, cisplatin and doxorubicin over a broad range of concentrations in human colon cancer cells and NSCLC cells. Synergy between CYC682 and gemcitabine strongly suggests that, despite being closely related, these two compounds have distinct mechanisms of action in cancer cells.
Various modifications and variations of the invention will be apparent to those skilled in the art. The described modes for carrying out the invention are claimed herein.

**Table 1: Antiproliferative effects ($IC_{50}$s using MTT assay) of CYC682, CNDAC, and CYC202 and several anticancer drugs in our panel of human cancer cell lines**

| | IC50s (μM) | | | | | | | | | |
|---|---|---|---|---|---|---|---|---|---|---|
| | **CYC682** | **CNDAC** | **CYC202** | **Doxorubicin** | **Docetaxel** | **Cisplatin** | **Oxaliplatin** | **Ara-C** | **5-FU** | **Gemcitabine** |
| | **48 hours** | **48 hours** | **24 hours** | **24 hours** | **24 hours** | **24 hours** | **24 hours** | **24 hours** | **24 hours** | **24 hours** |
| **HT29** | 4.5 ± 0.7 | 160 ± 43 | 19 ± 3 | 1.07 ± 0.16 | 0.01 ± 0.002 | 25 ± 3 | 60 ± 12 | 130 ± 40 | 24 ± 1 | 0.015 ± 0.003 |
| **HCT116** | 3.0 ± 0.6 | 2.8 + 0.6 | 11 ± 2 | 0.10 ± 0.02 | 0.01 ± 0.02 | 9.2 ± 0.9 | 20 ± 4 | 1.2 ± 0.4 | 5.3 ± 1.3 | 0.05 ± 0.01 |
| **HCC2998** | 3.5 ± 0.8 | 110 ± 21 | 23 ± 5 | 0.70 ± 0.14 | 0.004 ± 0.001 | 25 ± 5 | 4 ± 1.4 | 3.7 ± 0.5 | 10 ± 1 | 0.01 ± 0.002 |
| **COLO205** | 5.0 ± 0.8 | 8 ± 2 | 12 ± 3 | 1.5 ± 0.5 | 0.006 ± 0.001 | 9.5 ± 2.5 | 8 ± 1.6 | 28 ± 8 | 240 ± 20 | 0.2 ± 0.1 |
| **MCF7** | 7.0 ± 1.8 | 850 ± 60 | 16 ± 3 | 0.7 ± 0.1 | 0.01 ± 0.004 | 32 ± 2 | 35 ± 7 | >300 | 10 ± 1 | 0.01 ± 0.002 |
| **MDA-MB-435** | 67 ± 14 | 95 ± 13 | 19 ± 4 | 2.0 ± 0.4 | 0.01 ± 0.002 | 13 ± 2.6 | 37 ± 21 | 38 ± 9 | 10 ± 2 | 0.04 ± 0.008 |
| **HOP62** | 6.0 ± 1.7 | 7.0 ± 1.8 | 8 ± 2 | 0.14 ± 0.04 | 0.02 ± 0.04 | 9.3 ± 0.9 | 200 ± 40 | 6.3 ± 2.8 | 118 ± 32 | 0.01 ± 0.002 |
| **HOP92** | 38 ± 8 | 23 ± 6 | 26 ± 4 | 0.10 ± 0.05 | 0.005 ± 0.001 | 4.4 ± 0.4 | 1.4 ± 0.2 | 1.3 ± 0.3 | 11 ± 1 | 0.02 ± 0.004 |
| **IGROVI** | 6.5 ± 1.6 | 89 ± 12 | 38 ± 4 | 1.3 ± 0.3 | 0.02 ± 0.01 | 26 ± 6 | 107 ± 21 | 89 ± 21 | 29 ± 1 | 0.02 ± 0.004 |
| **OVCAR1** | 45 ± 7 | >900 | 20 ± 6 | 1.0 ± 0.2 | 50 ± 0.12 | 12.10 ± 3.93 | 50 ± 10 | >300 | 24 ± 1 | 1.2 ± 0.2 |

**Tables 3 and 4: Summary of results obtained with CNDAC in combination with various cytotoxic agents.** Cells were treated with CNDAC in combination with the indicated cytotoxic agents using three different treatment regimes, as described in the Examples section. Results are the average of at least three independent experiments.

**Table 3**

| | | CNDAC pretreatment | | | Other pretreatment | | | Concomitant | | |
|---|---|---|---|---|---|---|---|---|---|---|
| Compound | Cell line | **ED50** | **ED75** | **ED90** | **ED50** | **ED75** | **ED90** | **ED50** | **ED75** | **ED90** |
| *Doxorubicin | H1299 | 0.95 | 1.24 | 2.23 | 0.64 | 0.83 | 1.63 | 0.63 | 1.14 | 2.75 |
| Cisplatin | H1299 | 1.16 | 1.5 | 13.68 | 0.65 | 0.93 | 1.96 | 0.77 | 1.65 | 7.56 |
| *Gemcitabine | H1299 | | | | | | | 0.65 | 1.83 | 6.11 |
| Docetaxel | H1299 | | | | | | | 0.93 | 1.13 | 2.01 |
| Docetaxel | RERF-LC-MA | | | | | | | 0.9 | 0.97 | 1.1 |

In Table 3, combinations of CNDAC and cytotoxic agents marked with an asterisk (*) are included as reference examples only.

**Table 4**

| | | CNDAC pretreatment | | | Vinorelbine pretreatment | | | Concomitant | | |
|---|---|---|---|---|---|---|---|---|---|---|
| Compound | Cell line | **ED50** | **ED75** | **ED90** | **ED50** | **ED75** | **ED90** | **ED50** | **ED75** | **ED90** |
| Vinorelbine | H1299 | 1.04 | 0.67 | 0.67 | 0.60 | 0.56 | 0.66 | 0.63 | 0.97 | 2.25 |

## Claims

1. A combination comprising 2'-cyano-2'-deoxy-$N^4$-palmitoyl-1-β-D-arabinofuranosyl-cytosine, or a metabolite thereof which is 1-(2-C-Cyano-2-deoxy-β-D-arabino-pentafuranosyl)-cytosine, or a pharmaceutically acceptable salt thereof, and a cytotoxic agent selected from: (a) vinorelbine; (b) docetaxel; and (c) a platinum antineoplastic agent selected from cisplatin and oxaliplatin.

2. A combination according to claim 1 wherein the cytotoxic agent is vinorelbine.

3. A combination according to claim 1 wherein the cytotoxic agent is docetaxel.

4. A combination according to claim 1 wherein the platinum antineoplastic agent is cisplatin.

5. A combination according to claim 1 wherein the platinum antineoplastic agent is oxaliplatin.

6. A combination according to claim 1 or claim 2 which comprises 1-(2-C-Cyano-2-deoxy-β-D-arabino-pentafuranosyl)-cytosine and vinorelbine.

7. A combination according to claim 1 or claim 4 which comprises 1-(2-C-Cyano-2-deoxy-β-D-arabino-pentafuranosyl)-cytosine and cisplatin.

8. A combination according to any preceding claim which further comprises a pharmaceutically acceptable carrier, diluent or excipient.

9. Use of a combination according to any one of claims 1 to 8 in the preparation of a medicament for treating a proliferative disorder.

10. Use of 2'-cyano-2'-deoxy-$N^4$-palmitoyl-1-β-D-arabinofuranosyl-cytosine, or a metabolite thereof which is 1-(2-C-Cyano-2-deoxy-β-D-arabino-pentafuranosyl)-cytosine, or a pharmaceutically acceptable salt thereof, in the preparation of a medicament for the treatment of a proliferative disorder, wherein said treatment comprises simultaneously, sequentially or separately administering a cytotoxic agent selected from (a) vinorelbine; (b) docetaxel; and (c) a platinum antineoplastic agent selected from cisplatin and oxaliplatin, to a subject.

**11.** Use according to claim 10 wherein the 2'-cyano-2'-deoxy-$N^4$-palmitoyl-1-β-D-arabinofuranosyl-cytosine, or metabolite thereof according to claim 10, and the cytotoxic agent as defined in claim 10 are to be administered simultaneously.

**12.** Use according to claim 10 wherein the 2'-cyano-2'-deoxy-$N^4$-palmitoyl-1-β-D-arabinofuranosyl-cytosine, or metabolite thereof according to claim 10, and the cytotoxic agent as defined in claim 10, are to be administered sequentially or separately.

**13.** Use according to claim 12 wherein the cytotoxic agent as defined in claim 10 is to be administered sequentially or separately prior to the 2'-cyano-2'-deoxy-$N^4$-palmitoyl-1-β-D-arabinofuranosyl-cytosine, or metabolite thereof according to claim 10.

**14.** Use according to claim 12 wherein the 2'-cyano-2'-deoxy-$N^4$-palmitoyl-1-β-D-arabinofuranosyl-cytosine, or metabolite according to claim 10 thereof, is to be administered sequentially or separately prior to the cytotoxic agent as defined in claim 10.

**15.** Use according to any one of claims 10 to 14 wherein the proliferative disorder is cancer.

**16.** Use according to claim 15 wherein the cancer is colon cancer.

**17.** A combination according to any one of claims 1 to 8 for use in treating a proliferative disorder.

**Patentansprüche**

**1.** Kombination umfassend 2'-Cyano-2'-desoxy-$N^4$-palmitoyl-1-β-D-arabinofuranosyl-Cytosin oder einen Metaboliten davon, welcher 1-(2-C-Cyano-2-desoxy-β-D-arabino-pentafuranosyl)-Cytosin ist, oder ein pharmazeutisch verträgliches Salzes davon und ein zytotoxisches Mittel, das ausgewählt ist unter: (a) Vinorelbin, (b) Docetaxel und (c) einem antineoplastischen Platinmittel, ausgewählt unter Cisplatin und Oxaliplatin.

**2.** Kombination nach Anspruch 1, wobei das zytotoxische Mittel Vinorelbin ist.

**3.** Kombination nach Anspruch 1, wobei das zytotoxische Mittel Docetaxel ist.

**4.** Kombination nach Anspruch 1, wobei das antineoplastische Platinmittel Cisplatin ist.

**5.** Kombination nach Anspruch 1, wobei das antineoplastische Platinmittel Oxaliplatin ist.

**6.** Kombination nach Anspruch 1 oder Anspruch 2, umfassend 1-(2-C-Cyano-2-desoxy-β-D-arabino-pentafuranosyl)-Cytosin und Vinorelbin.

**7.** Kombination nach Anspruch 1 oder Anspruch 4, umfassend 1-(2-C-Cyano-2-desoxy-β-D-arabino-pentafuranosyl)-Cytosin und Cisplatin.

**8.** Kombination nach einem der vorangehenden Ansprüche, welche weiterhin einen pharmazeutisch verträglichen Träger, Verdünnungsstoff oder Hilfsstoff umfasst.

**9.** Verwendung einer Kombination nach einem der Ansprüche 1 bis 8 bei der Herstellung eines Arzneimittels zur Behandlung einer proliferativen Störung.

**10.** Verwendung von 2'-Cyano-2'-desoxy-$N^4$-palmitoyl-1-β-D-arabinofuranosyl-Cytosin oder eines Metaboliten davon, welcher 1-(2-C-Cyano-2-desoxy-β-D-arabino-pentafuranosyl)-Cytosin ist, oder eines pharmazeutisch verträglichen Salzes davon bei der Herstellung eines Arzneimittels für die Behandlung einer proliferativen Störung, wobei die Behandlung die gleichzeitige, aufeinanderfolgende oder separate Verabreichung eines zytotoxischen Mittels, das unter (a) Vinorelbin, (b) Docetaxel und (c) einem antineoplastischen Platinmittel, ausgewählt unter Cisplatin und Oxaliplatin, ausgewählt ist, an ein Subjekt.

**11.** Verwendung nach Anspruch 10, wobei das 2'-Cyano-2'-desoxy-$N^4$-palmitoyl-1-β-D-arabinofuranosyl-Cytosin oder

ein Metabolit davon gemäß Anspruch 10 und das zytotoxische Mittel, wie es in Anspruch 10 definiert ist, gleichzeitig verabreicht werden.

**12.** Verwendung nach Anspruch 10, wobei das 2'-Cyano-2'-desoxy-$N^4$-palmitoyl-1-β-D-arabinofuranosyl-Cytosin oder ein Metabolit davon gemäß Anspruch 10 und das zytotoxische Mittel, wie es in Anspruch 10 definiert ist, aufeinanderfolgend oder separat verabreicht werden.

**13.** Verwendung nach Anspruch 12, wobei das zytotoxische Mittel, wie es in Anspruch 10 definiert ist, in einer Abfolge oder separat vor dem 2'-Cyano-2'-desoxy-$N^4$-palmitoyl-1-β-D-arabinofuranosyl-Cytosin oder einem Metaboliten davon, wie er in Anspruch 10 definiert ist, verabreicht werden soll.

**14.** Verwendung nach Anspruch 12, wobei das 2'-Cyano-2'-desoxy-$N^4$-palmitoyl-1-β-D-arabinofuranosyl-Cytosin oder ein Metabolit davon gemäß Anspruch 10 in einer Abfolge oder separat vor dem zytotoxischen Mittel, wie es in Anspruch 10 definiert ist, verabreicht werden soll.

**15.** Verwendung nach einem der Ansprüche 10 bis 14, wobei die proliferative Störung Krebs ist.

**16.** Verwendung nach Anspruch 15, wobei der Krebs Darmkrebs ist.

**17.** Kombination nach einem der Ansprüche 1 bis 8 für die Verwendung bei der Behandlung einer proliferativen Störung.

**Revendications**

**1.** Combinaison comprenant de la 2'-cyano-2'-désoxy-$N^4$-palmitoyl-1-β-D-arabinofuranosyl-cytosine, ou un métabolite de celle-ci qui est une 1-(2-C-cyano-2-désoxy-β-D-arabino-pentafuranosyl)-cytosine, ou un sel pharmaceutiquement acceptable de celle-ci, et un agent cytotoxique choisi parmi : (a) la vinorelbine ; (b) le docétaxel ; et (c) un agent antinéoplasique à base de platine choisi parmi le cisplatine et l'oxaliplatine.

**2.** Combinaison selon la revendication 1, dans laquelle l'agent cytotoxique est la vinorelbine.

**3.** Combinaison selon la revendication 1, dans laquelle l'agent cytotoxique est le docétaxel.

**4.** Combinaison selon la revendication 1, dans laquelle l'agent antinéoplasique à base de platine est le cisplatine.

**5.** Combinaison selon la revendication 1, dans laquelle l'agent antinéoplasique à base de platine est l'oxaliplatine.

**6.** Combinaison selon la revendication 1 ou la revendication 2, qui comprend de la 1-(2-C-cyano-2-désoxy-β-D-arabino-pentafuranosyl)-cytosine et de la vinorelbine.

**7.** Combinaison selon la revendication 1 ou la revendication 4, qui comprend de la 1-(2-C-cyano-2-désoxy-p-D-arabino-pentafuranosyl)-cytosine et du cisplatine.

**8.** Combinaison selon l'une quelconque des revendications précédentes, qui comprend en outre un véhicule, un diluant ou un excipient pharmaceutiquement acceptable.

**9.** Utilisation d'une combinaison selon l'une quelconque des revendications 1 à 8 dans la préparation d'un médicament pour traiter un trouble prolifératif.

**10.** Utilisation de la 2'-cyano-2'-désoxy-$N^4$-palmitoyl-1-β-D-arabinofuranosylcytosine, ou d'un métabolite de celle-ci qui est une 1-(2-C-cyano-2-désoxy-β-D-arabino-pentafuranosyl)-cytosine, ou d'un sel pharmaceutiquement acceptable de celle-ci, dans la préparation d'un médicament pour le traitement d'un trouble prolifératif, dans laquelle ledit traitement comprend simultanément, de manière séquentielle ou séparément, l'administration d'un agent cytotoxique choisi parmi : (a) la vinorelbine ; (b) le docétaxel ; et (c) un agent antinéoplasique à base de platine choisi parmi le cisplatine et l'oxaliplatine, à un sujet.

**11.** Utilisation selon la revendication 10, dans laquelle la 2'-cyano-2'-désoxy-$N^4$-palmitoyl-1-β-D-arabinofuranosyl-cytosine, ou un métabolite de celle-ci selon la revendication 10, et l'agent cytotoxique défini dans la revendication 10

doivent être administrés simultanément.

**12.** Utilisation selon la revendication 10, dans laquelle la 2'-cyano-2'-désoxy-$N^4$-palmitoyl-1-β-D-arabinofuranosyl-cytosine, ou un métabolite de celle-ci selon la revendication 10, et l'agent cytotoxique défini dans la revendication 10 doivent être administrés de manière séquentielle ou séparément.

**13.** Utilisation selon la revendication 12, dans laquelle l'agent cytotoxique défini dans la revendication 10 doit être administré de manière séquentielle ou séparément avant la 2'-cyano-2'-désoxy-$N^4$-palmitoyl-1-β-D-arabinofuranosyl-cytosine, ou un métabolite de celle-ci selon la revendication 10.

**14.** Utilisation selon la revendication 12, dans laquelle la 2'-cyano-2'-désoxy-$N^4$-palmitoyl-1-β-D-arabinofuranosyl-cytosine, ou un métabolite de celle-ci selon la revendication 10, doit être administré de manière séquentielle ou séparément avant l'agent cytotoxique défini dans la revendication 10.

**15.** Utilisation selon l'une quelconque des revendications 10 à 14, dans laquelle le trouble prolifératif est un cancer.

**16.** Utilisation selon la revendication 15, dans laquelle le cancer est le cancer du colon.

**17.** Combinaison selon l'une quelconque des revendications 1 à 8 destinée à une utilisation dans le traitement d'un trouble prolifératif.

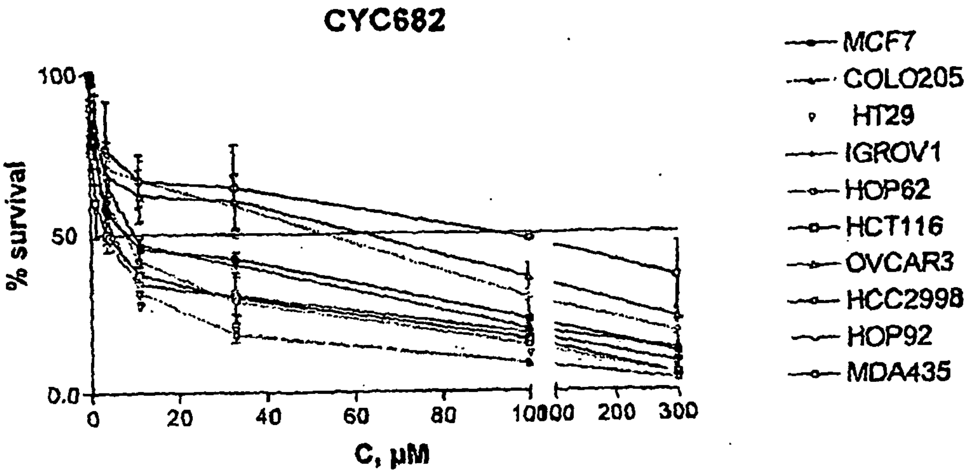
CYC682
100
50
0.0
% survival
0
20
40
60
80
100
100
200
300
C, µM
MCF7
COLO205
HT29
IGROV1
HOP62
HCT116
OVCAR3
HCC2998
HOP92
MDA435

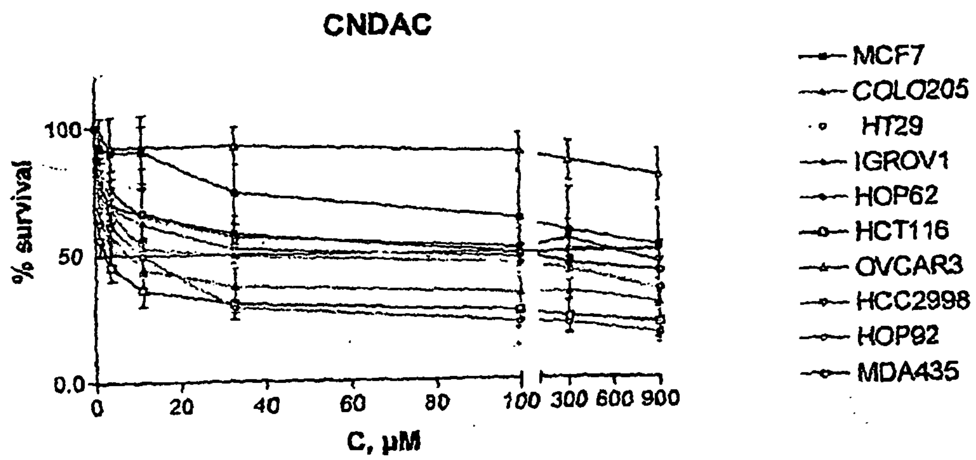
CNDAC
100
50
0.0
% survival
0
20
40
60
80
100
300
600
900
C, µM
MCF7
COLO205
HT29
IGROV1
HOP62
HCT116
OVCAR3
HCC2998
HOP92
MDA435

FIGURE 1

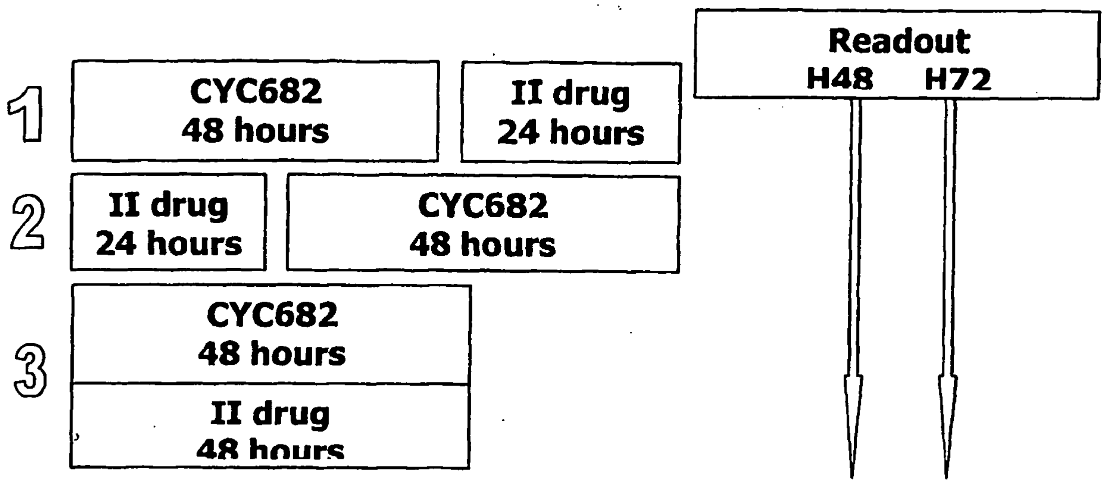
Readout
H48 H72
1
CYC682
48 hours
II drug
24 hours
2
II drug
24 hours
CYC682
48 hours
3
CYC682
48 hours
II drug
48 hours

FIGURE 2

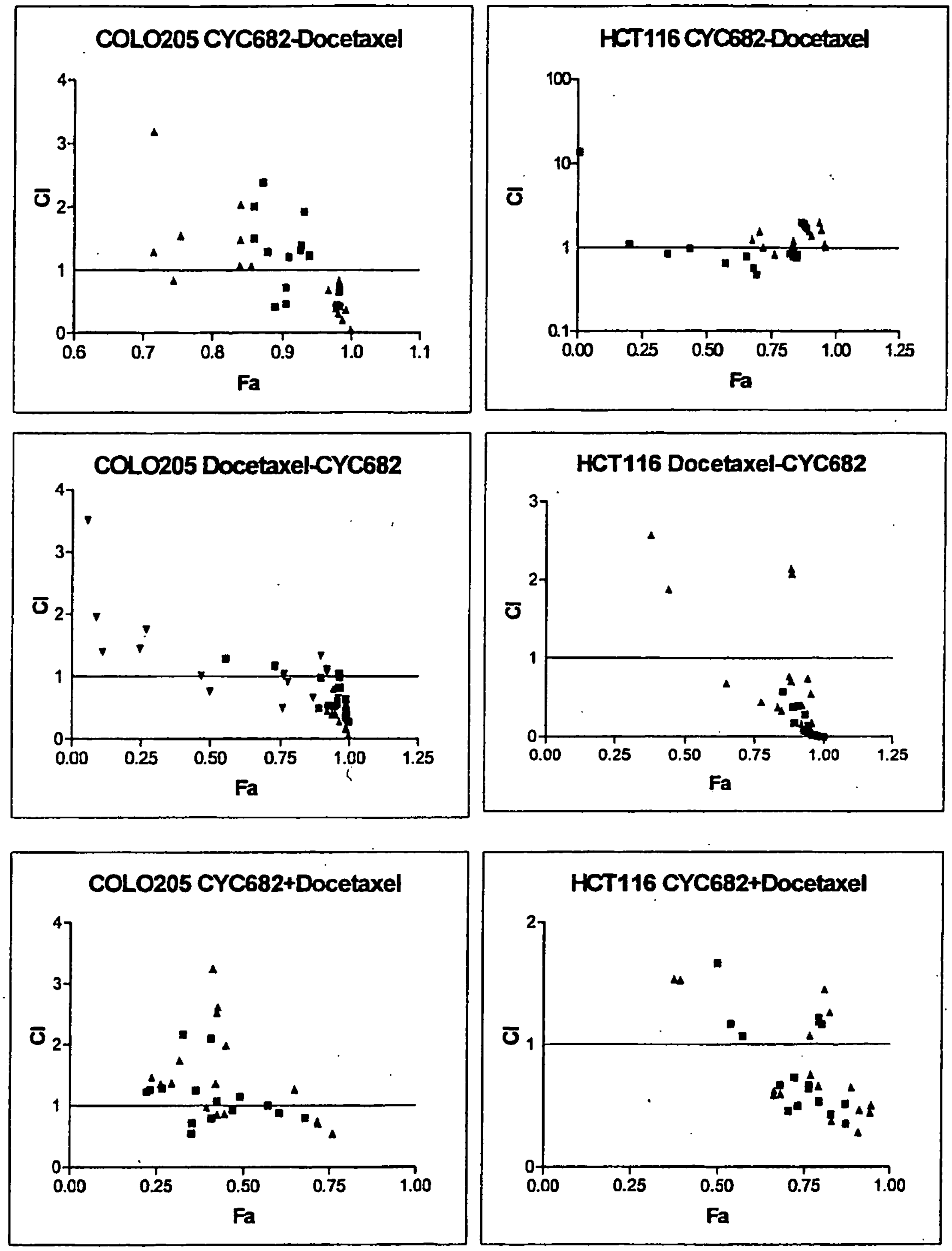
COLO205 CYC682-Docetaxel
HCT116 CYC682-Docetaxel
COLO205 Docetaxel-CYC682
HCT116 Docetaxel-CYC682
COLO205 CYC682+Docetaxel
HCT116 CYC682+Docetaxel
CI
Fa

FIGURE 3

(Reference)

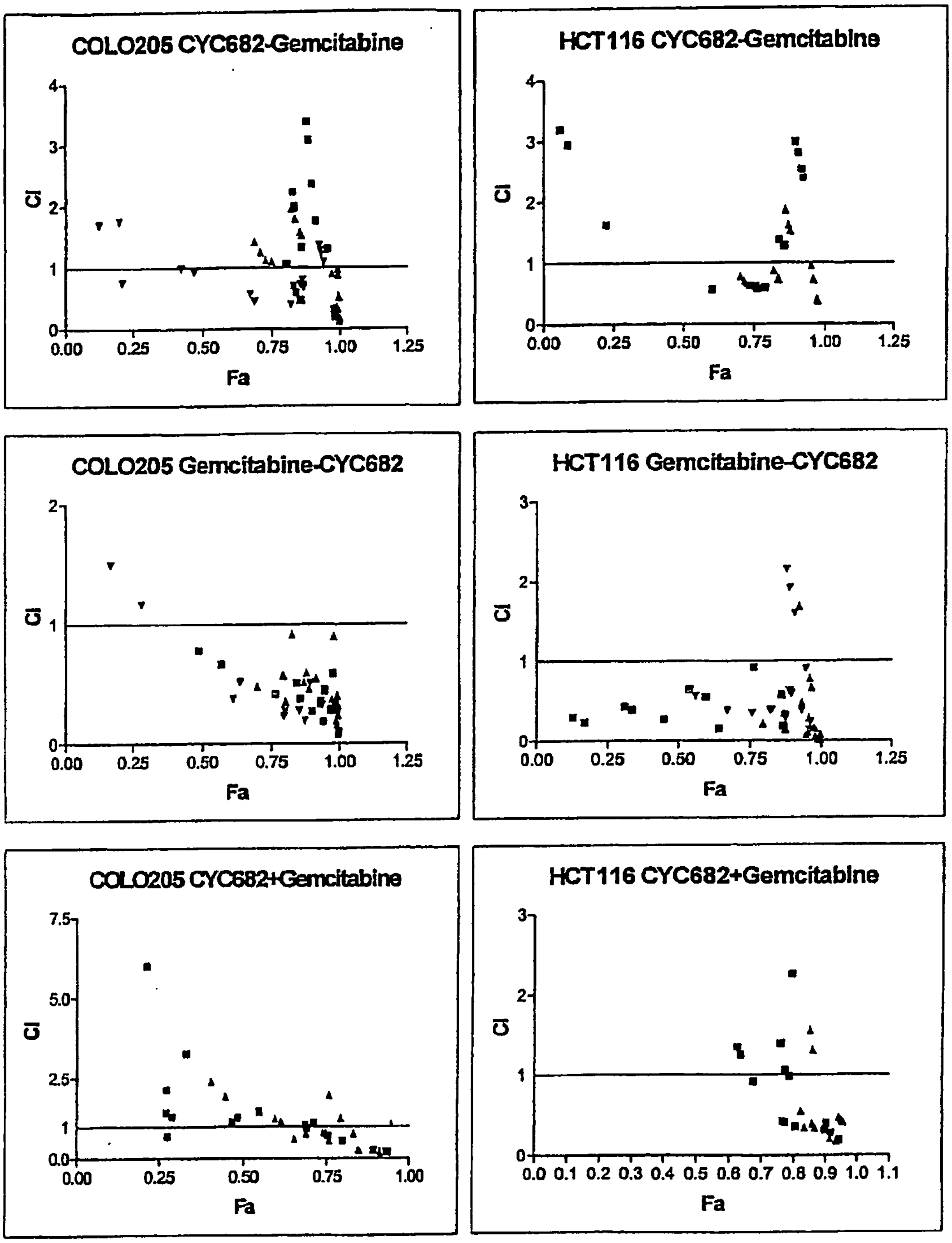


FIGURE 4

(Reference)

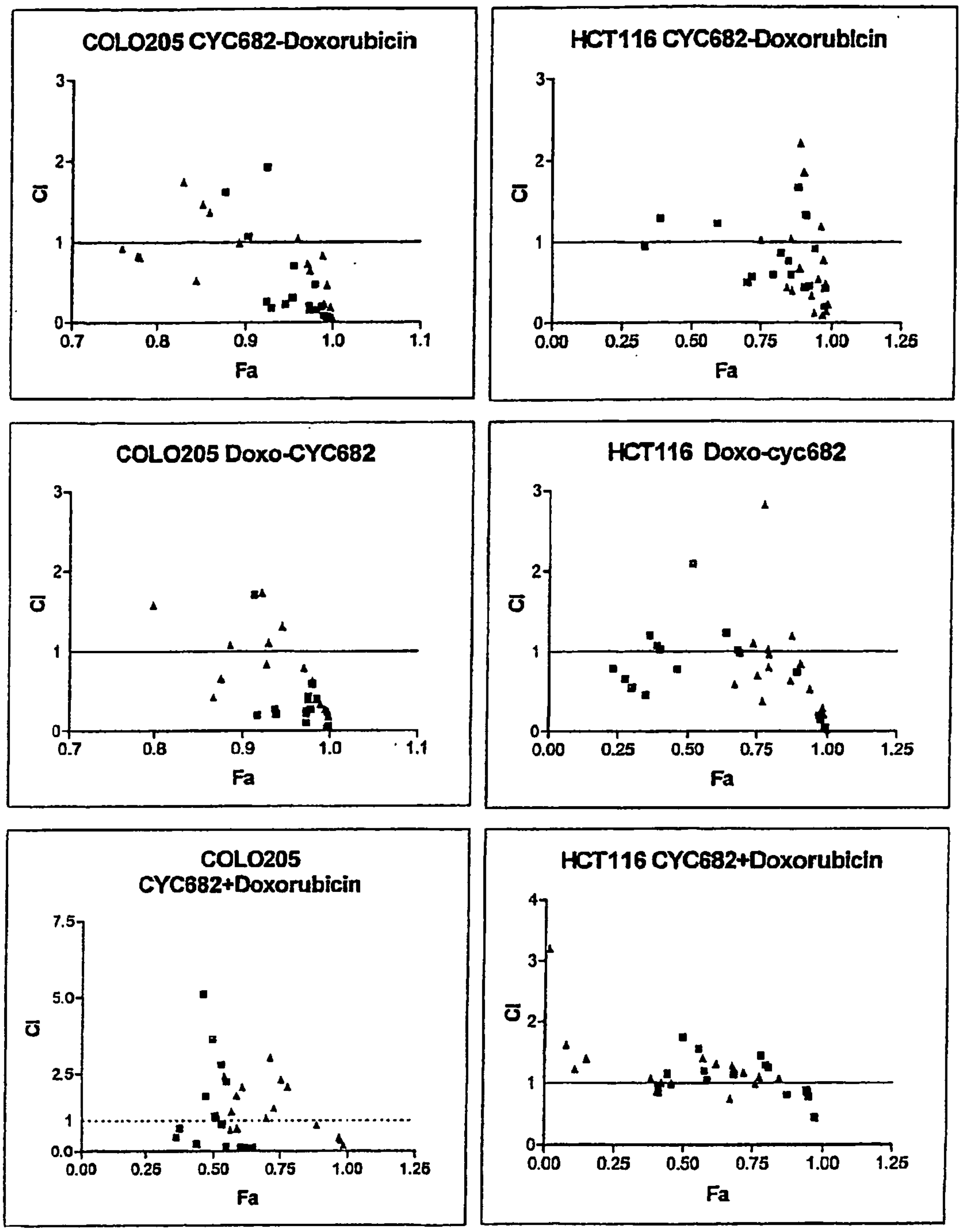


FIGURE 5

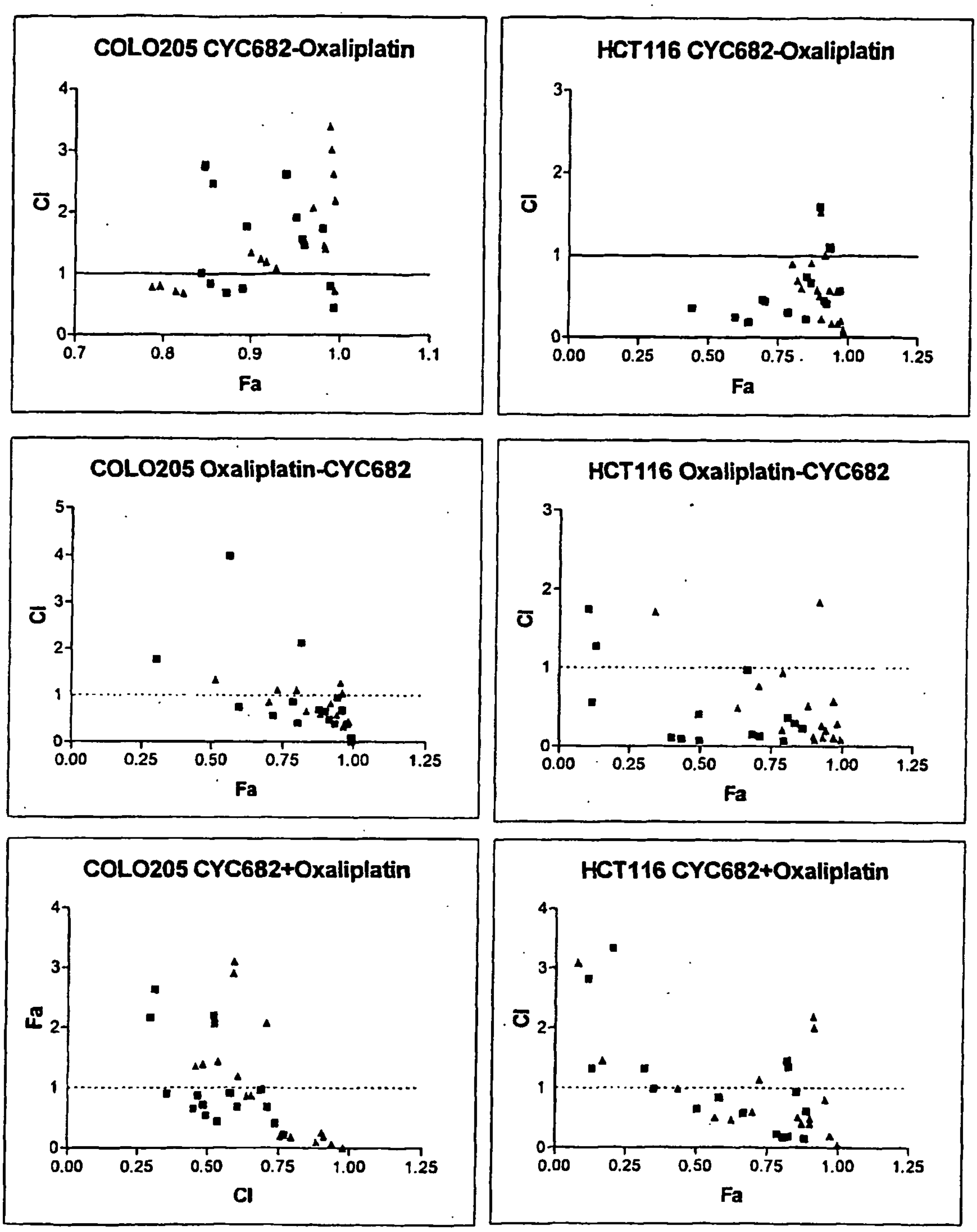

COLO205 CYC682-Oxaliplatin
CI
Fa
HCT116 CYC682-Oxaliplatin
CI
Fa
COLO205 Oxaliplatin-CYC682
CI
Fa
HCT116 Oxaliplatin-CYC682
CI
Fa
COLO205 CYC682+Oxaliplatin
Fa
CI
HCT116 CYC682+Oxaliplatin
CI
Fa


FIGURE 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 536936 A
- WO 2005053699 A


### Non-patent literature cited in the description


- **Duschinsky, R. et al.** *J. Am. Chem. Soc.,* 1957, vol. 79, 4559
- **Hiller, SA. et al.** *Dokl. Akad. Nauk USSR,* 1967, vol. 176, 332
- **Fujii, S. et al.** *Gann,* 1978, vol. 69, 763
- **Hoshi, A. et al.** *Gann,* 1976, vol. 67, 725
- **Cook, A. F. et al.** *J. Med. Chem.,* 1979, vol. 22, 1330
- **Evance, J. S. et al.** *Proc. Soc. Exp. Bio. Med.,* 1961, vol. 106, 350
- **Hoshi, A et al.** *Gann,* 1972, vol. 63, 353
- **Aoshima, M. et al.** *Cancer Res.,* 1976, vol. 36, 2726
- **Galmarini et al.** Nucleoside analogues and nucleobases in cancer treatment. *Lancet Oncol.,* July 2002, vol. 3 (7), 415-24
- **Galmarini et al.** In vivo mechanisms of resistance to cytarabine in acute myeloid leukaemia. *Br J Haematol.,* June 2002, vol. 117 (4), 860-8
- **Hanaoka, K. et al.** *Int. J. Cancer,* 1999, vol. 82, 226-236
- **Donehower R et al.** *Proc Am Soc Clin Oncol,* 2000
- **Burch, PA et al.** *Proc Am Soc Clin Oncol,* 2001
- **Wu M et al.** *Cancer Research,* 2003, vol. 63, 2477-2482
- **Wiseman LR ; Adkins JC ; Plosker GL et al.** Oxaliplatin: a review of its use in the management of metastatic colorectal cancer. *Drugs Aging,* 1999, vol. 14 (6), 459-75
- **Misset JL ; Bleiberg H ; Sutherland W et al.** Oxaliplatin Clinical Activity: A Review. *Critical Reviews in Oncology-Hematology,* 2000, vol. 35 (2), 75-93
- **Cvitkovic E ; Bekradda M.** Oxaliplatin: A New Therapeutic Option in Colorectal Cancer. *Semin Oncol,* 1999, vol. 26 (6), 647-62
- **Freyer G ; Bossard N ; Romestaing P et al.** Oxaliplatin (OXA), 5-fluorouracil (5FU), L-folinic acid (FA) and concomitant irradiation in patients with rectal cancer: A phase 1 study. *Proc Am Soc Clin Oncol,* 2000, vol. 19, 260a
- **Carraro S ; Roca E ; Cartelli C et al.** Oxaliplatin (OXA), 5-fluorouracil (5-Fu) and leucovorin (LV) plus radiotherapy in unresectable rectal cancer (URC): Preliminary results. *Proc Am Soc Clin Oncol,* 2000, vol. 19, 291a
- Handbook of Pharmaceutical Excipients. 1994
- **Berge et al.** *J Pharm Sci,* 1977, vol. 66, 1-19
- **Hansen MB ; Nielsen SE ; Berg K.** Re-examination and further development of a precise and rapid dye method for measuring cell growth/cell kill. *J Immunol Methods,* 12 May 1989, vol. 119 (2), 203-10
- **Mosmann, T.** Rapid colorimetric assay for cellular growth and survival: application to proliferation and cytotoxicity assays. *J Immunol Methods,* 16 December 1983, vol. 65 (1-2), 55-63
- **Chou TC ; Talalay P.** Quantitative analysis of dose-effect relationships: the combined effects of multiple drugs or enzyme inhibitors. *Adv Enzyme Regul.,* 1984, vol. 22, 27-55